# EUROPEAN PATENT APPLICATION

(11) **EP 1 050 285 A1**
(43) Date of publication of application: **08.11.2000**
(21) Application number: 00109474.7
(22) Date of filing: 04.05.2000
(51) Int. Cl.: A61F 5/055, A61F 5/01

(54) **Adjustable cervical collar**

(30) Priority: 05.05.1999 IT VE990016 U
(71) Applicant: Forner, Flavio, 31030- Covolo Di Pederobba (IT)
(72) Inventor: Forner, Flavio, 31030- Covolo Di Pederobba (IT)
(74) Representative: Piovesana, Paolo

(57) **Abstract**

A collar characterised by being formed from a plurality of side-by-side segments (2,8), of which the degree of yielding can be individually adjusted.

## Description

This invention relates to an adjustable collar.

In orthopedics and physiotherapy it is known to use rigid semirigid and soft collars, applied to the patient's neck to immobilize the neck and at the same time support the head in the case of traumatic lesions, operation-derived lesions or vertebral collapse. However the use of such known collars, even for short time period, produces muscular deficiency which in some cases can lead to atrophy.

According to the invention, this problem is eliminated by a collar as described in claim 1.

Some embodiments of the invention are described in detail hereinafter with reference to the accompanying drawings, on which:
- Figure 1: is a schematic perspective view of a first embodiment of the collar of the invention with its pockets upperly open;
- Figure 2: shows the same view of the first embodiment as Figure 1 but with its pockets closed;
- Figure 3: shows a first variant of the invention; and
- Figure 4: shows a second variant of the invention.

As can be seen from the figures, the collar of the invention consists of a belt with pockets which can be individually adjusted to vary the physical characteristics of that pocket.

The collar is hence in the form of a succession of segments of variable yieldability, to satisfy the particular function which the collar is to perform.

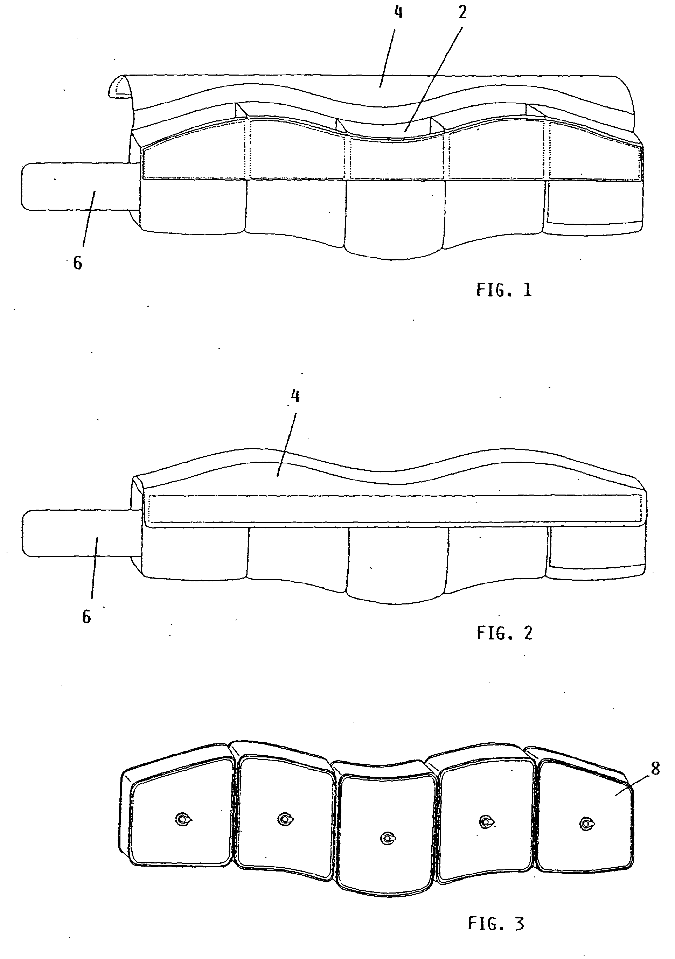

The collar shown in Figures 1 and 2 consists of a succession of pockets 2 provided with an upper aperture and associated with a single closure flap 4, common to all the pockets.

The closure is made stable by using traditional self-adhesive systems, for example of Velcro® type.

A fastening web 8, also provided with Velcro®, is applied to the succession of pockets 2, to adjustably apply the collar to the patient's neck.

Fillers of different characteristics and in particular of different yieldability can be inserted into each pocket 2 or into the desired number of pockets 2. More specifically, polyurethane blocks, or bags of high thermal capacity gel, can be inserted into the pockets 2.

In the embodiment shown in Figure 3, in which for representational simplicity the collar attachment means are not shown, the individual segments forming the collar consist of air-containing pockets 8, each provided with a blowing port 10 allowing them to be inflated by the user to the desired extent.

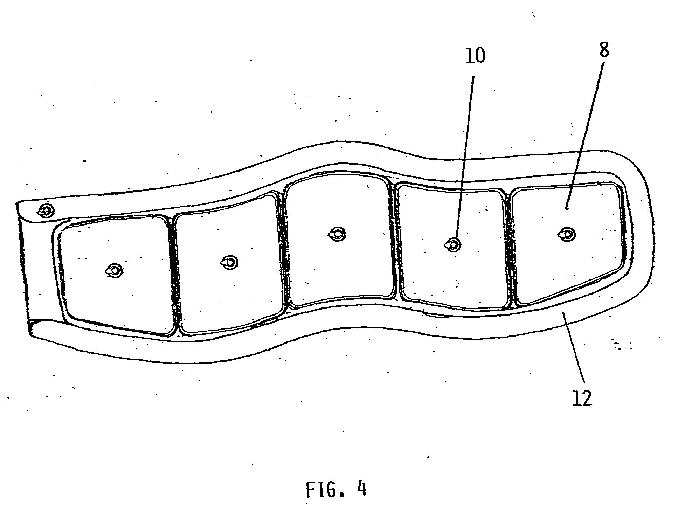

In the embodiment shown in Figure 4, the pockets 8 have an inflatable air chamber 12 along their upper and lower edge.

In a further embodiment, not shown on the drawings, each segment can consist of a inflatable pocket, to the outside of which there can be applied, for example by Velcro®, a further pocket containing a gel bag or a polyurethane block.

Whatever the embodiment adopted, the collar of the invention is used in the manner of traditional collars, ie to support the head while maintaining the collar clamped in the correct position.

However in contrast to traditional collars, the collar of the invention also enables the neck to be only partially clamped, ie only the painful or injured part thereof, so preventing any muscular deficiency to that part of the neck not requiring immobilization.

In addition, if gel bags are used, the temperature to which the gel has been adjusted can be transmitted to that part of the neck to which they are applied.

Besides offering the same support as a traditional collar, the collar of the invention is particularly advantageous, in that:
- it enables the degree of yieldability to be adjusted locally, so providing support only where effectively necessary, and
- the support function can be augmented with or replaced by hot and/or cold therapy.

## Claims

1. A collar characterised by being formed from a plurality of side-by-side segments (2,8), of which the degree of yielding can be individually adjusted.

2. A collar as claimed in claim 1, characterised in that the segments consist of pockets (2) provided with an upper aperture and closure flap (4), and housing fillers of different characteristics.

3. A collar as claimed in claim 2, characterised in that all the pockets are associated with a single closure flap.

4. A collar as claimed in claim 1, characterised by comprising a fastening web (6).

5. A collar as claimed in claim 4, characterised in that the web (6) consists of Velcro®.

6. A collar as claimed in claim 2, characterised in that the fillers have different degrees of yieldability.

7. A collar as claimed in claim 6, characterised in that the fillers consists of gel of high thermal capacity.

8. A collar as claimed in claim 6, characterised in that the fillers consists of polyurethane blocks.

9. A collar as claimed in claim 1, characterised in that each segment consists of an inflatable impermeable pocket (8).

10. A collar as claimed in claim 1, characterised in that each segment consists of an inflatable impermeable pocket to which a further pocket with a filler is applied.

11. A collar as claimed in claim 2, characterised in that at least one edge extending along all the pockets is formed of soft material.

12. A collar as claimed in claim 11, characterised in that said edge consists of an air chamber (12).

13. A collar as claimed in claim 12, characterised in that the air chambers are inflatable.
